# EUROPEAN PATENT SPECIFICATION

(11) **EP 0 632 835 B1**
(45) Date of publication and mention of the grant of the patent: **28.02.2001**
(21) Application number: 92920421.2
(22) Date of filing: 24.09.1992
(51) Int. Cl.: C12N 15/82, C12N 15/54, A01H 5/00, A01N 63/00

(54) **TRANSGENIC PLANTS DISPLAYING MULTIPLE VIRUS RESISTANCE AND A PROCESS FOR THEIR PRODUCTION**
Multiple Virus-Resistenz aufweisende transgene Pflanzen und Verfahren zu ihrer Herstellung
PLANTES TRANSGENIQUES PRESENTANT UNE RESISTANCE A UNE PLURALITE DE VIRUS ET LEUR PROCEDE DE PRODUCTION

(30) Priority: 18.03.1992 EP 92104676
(43) Date of publication of application: 11.01.1995
(73) Proprietor: Truve, Erkki, 10311 Tallinn (EE); Kelve, Merike, 10114 Tallinn (EE); Teeri, Teemu, 02180 Espoo (FI); Saarma, Mart, 00570 Helsinki (FI)
(72) Inventor: Truve, Erkki, 10311 Tallinn (EE); Kelve, Merike, 10114 Tallinn (EE); Teeri, Teemu, 02180 Espoo (FI); Saarma, Mart, 00570 Helsinki (FI)
(74) Representative: VOSSIUS & PARTNER
(86) International application number: EP9202217
(87) International publication number: WO9319187

(56) References cited:
- CIBA FOUNDATION SYMPOSIUM vol. 133, 1987, pages 109 - 119 SELA, I., ET AL. 'Resistance systems related to the N gene and their comparison with interferon' cited in the application
- FASEB JOURNAL vol. 4, no. 13, 1990, BETHESDA, MD US pages 3124 - 3130 SCHR\DER, H.C., ET AL. 'Protection of HeLa-T4+ cells against human immunodeficiency virus (HIV) infection after stable transfection with HIV LTR -2',5'- oligoadenylate synthetase hybrid gene'
- BIOLOGICAL ABSTRACTS vol. 91 , 1991, Philadelphia, PA, US; abstract no. 66022, BABOSHA, A.V., ET AL. 'Oligoadenylates and oligoadenylate synthetase of potato plants in defense reactions to a viral pathogen'
- BIOLOGICAL ABSTRACTS vol. 90 , 1990, Philadelphia, PA, US; abstract no. 106702, SHER, N., ET AL. 'Induction of an ATP-polymerizing enzyme in TMV-infected tobacco and its homology to the human 2' -5' -oligoadenylate synthetase'
- DEVASH, Y. ET AL.: "Plant oligoadenylates: Enzymatic synthesis, isolation and biological activities", BIOCHEMISTRY, , 1985, vol. 24, no. , pages 593 to 599
- DEVASH, Y. ET AL.: "5'-dephosphorylated 2', 5'-adenylate trimer and its analogs", THE JOURNAL OF BIOLOGICAL CHEMISTRY, , 1984, vol. 259, no. 6, pages 3482 to 3486

## Description

This invention relates to transgenic plants displaying multiple virus resistance which contain a genetically engineered DNA sequence encoding at least one polypeptide having a 2,5A synthetase activity. Moreover, this invention relates to a process for the production of said transgenic plants and to the use of said genetically engineered DNA sequence.

Several methods for the construction of virus-resistant plants are described in the state of the art. Genetically engineered resistance to a number of plant viruses has been reported by expressing coat protein of a respective plant virus in transgenic plants (Beachy et al., Annu. Rev. Phytopathol. 28 (1990), 451-474).
A substantial virus-resistance to several plant viruses has also been demonstrated by expression of specific viral antisense RNA in transgenic plants (Cuozzo et al, Bio/Technology 6 (1988), 549-557; Hemenway et al., EMBO J. 7 (1988), 1273-1280). The viral antisense RNA exhibits its function either by hybridizing to specific viral DNA or RNA sequences and thus blocking further reactions which are important for the virus propagation, or by ribozyme activity which results in a specific cleavage of viral RNA upon hydridisation to said viral RNA. The main drawback of the above mentioned methods for the construction of virus-resistant transgenic plants is that said transgenic plants are resistant to only one virus or a specific group of viruses.

The 2,5A oligoadenylate pathway is part of the antiviral response system induced by interferons in mammalian cells (Lengyel, Annu. Rev. Biochem. 51 (1982), 251-282). At least some components of the 2,5A pathway have been detected in organisms other than mammals such as birds, reptilia and amphibia, insects, yeasts and even bacteria (Stark et al., Nature 278 (1979), 471-473; Cayley et al., Biochem. Biophys. Res. Comm. 108 (1982), 1243-1250; Laurence et al., Proc. Natl. Acad. Sci. U.S.A. 81 (1984), 2322-2326).
In plants, an antiviral factor (AVF), the production of which is stimulated by virus infection, was partially purified and its gene appeared to be homologous to human β-interferon; Sela et al., in: Plant resistance to Viruses: Ciba Symposium, No. 133 (1987), 109-119. Treatment of tobacco mosaic virus (TMV)-infected tobacco protoplasts with human interferon led to the inhibition of TMV-replication; Sela, Methods in Enzymology, 119 (1986), 744-752. Moreover, it has been demonstrated that 2,5A can inhibit TMV replication in tobacco plants; Devash et al., J. Biol. Chem. 259 (1984), 3482-3486. DNA sequences homologous to human 2,5A synthetase were also found in tobacco genomic DNA; Sela (1987), supra.
However, a 2,5A dependent RNAse which is part of the 2,5A oligoadenylate pathway in mammals has not yet been reported in plants in the state of the art. This has led to the conclusion that 2,5A does not inhibit virus infection in plants via a 2,5A dependent endonuclease; Devash et al., Biochemistry 24 (1985), 593-599.

Furthermore, it has been reported that interferon produced in transgenic plants does not inhibit virus propagation; De Zoeten et al., Vivology 172 (1989), 213-222.

In summary, the prior art does not permit the conclusion that the 2,5A oligoadenylate pathway can be used as a basis for constructing transgenic plants displaying multiple virus resistance.

Thus, the technical problem of the present invention is to provide a transgenic plant displaying multiple virus resistance using parts of the 2,5A oligoadenylate pathway.

The solution to the above technical problem is achieved by providing the embodiments characterized in the claims.

One object of the present invention relates to a transgenic plant displaying multiple virus resistance which contains a genetically engineered DNA sequence encoding at least one polypeptide having a 2,5A synthetase activity, wherein said polypeptide upon expression is capable of activating an endonuclease causing degradation of viral RNA.
The term "multiple virus resistance" refers to transgenic plants which are substantially resistant to a variety of virus groups such as potex-, carla- and tobamoviruses.
The term "genetically engineered DNA sequence" refers to a DNA sequence which has been manipulated by genetic engineering methods such as recombinant DNA techniques.
The term "polypeptide having a 2,5 synthetase activity" refers to any polypeptide which is capable of enzymatically synthesizing 5'-dephosphorylated or phosphorylated 2,5-oligonucleotides with three or more adenosine residues such as 2,5A₃ which is a trimer of adenylate linked by 2',5'-phosphodiester bonds and is dephosphorylated at its 5'-end.
The term "endonuclease causing degradation of viral RNA" refers to a heterologous or homologous endonuclease which is capable of degrading viral RNA by enzymatic cleavage. Said endonuclease is activated by a 2,5A which is synthesized by a polypeptide having a 2,5A synthetase activity.

In a preferred embodiment of the present invention said DNA sequence or at least one part of said DNA sequence is homologous or heterologous to said transgenic plant.

In a further embodiment of the present invention said DNA sequence is a chimeric DNA sequence.

In a further preferred embodiment of the present invention said DNA sequence additionally encodes at least one selectable marker and/or at least one further polypeptide such as said endonuclease.

In a preferred embodiment of the present invention the DNA sequence encoding said polypeptide is derived from a mammalian gene, a plant gene or a microorganism gene or is a synthetic gene.

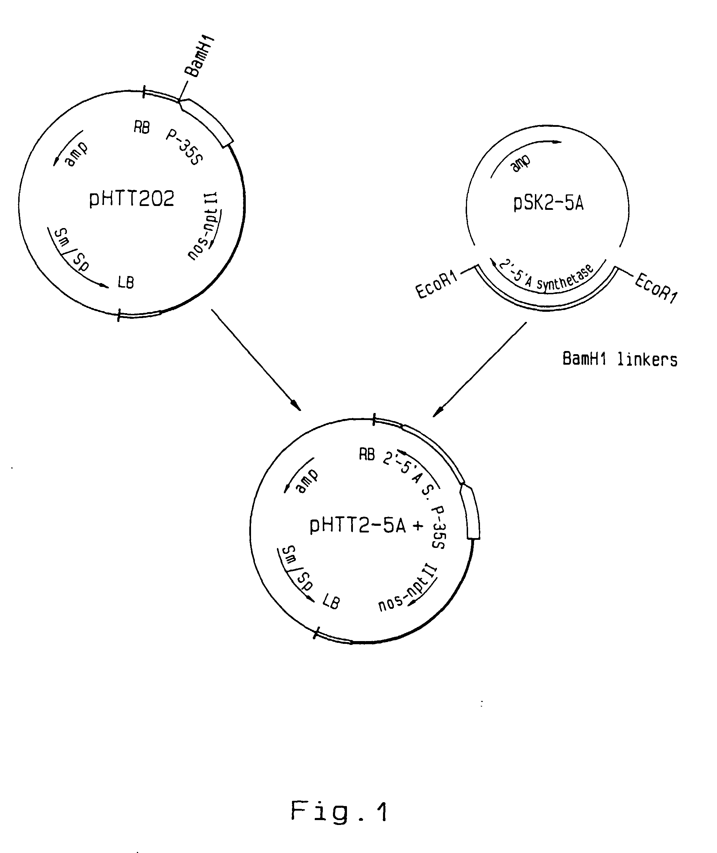

In a particularly preferred embodiment of the present invention the DNA sequence encoding said polypeptide is a rat 2,5A synthetase gene (see Fig. 1).

In a preferred embodiment of the present invention said endonuclease is a plant RNAse L.

The expression of said polypeptide is under the control of an inducible or a constitutive promoter functioning in plants and allowing an induced and/or increased expression of said polypeptide. Examples of such promoters are cauliflower mosaic virus 35S-promoter, rice actin promoter, rbc S promoter from different species, Agrobacter TR2' promoter, phaseolin gene promoter or the NOS promoter. In a preferred embodiment of the present invention the expression of the 2,5A synthesizing polypeptide is under the control of the cauliflower mosaic virus 35S-promoter.

The transgenic plant is a monocotyledoneous or a dicotyledoneous plant. In a preferred embodiment of the present invention the transgenic plant is tobacco, rice, wheat, barley, maize, tomato, cucumber, soya, sweet potato, grapes, rapeseed, sugar beat, cotton, tea, sunflower, strawberry, rose, chrysanthemum, sweet pepper or potato.

A further object of the present invention is a propagating material derived from a transgenic plant displaying multiple virus resistance.

The term "propagating material" refers to intact plants or differentiated or undifferentiated plant tissue such as root, stem, leaf, callus, protoplast, suspension cultures and seeds.

A further object of the present invention is a process for the production of a transgenic plant displaying multiple virus resistance, comprising the introduction of a genetically engineered DNA sequence encoding at least one polypeptide having a 2,5A synthetase activity, into the genetic material of a suitable plant.
The term "genetic material" refers to the nuclear genome of a plant cell, an organell genome of the plant cell or an extrachromosomal form.

The term "introduction" refers to a method which is capable of introducing said genetically engineered DNA sequence into said genetic material of a plant cell. Preferred examples of said method are Agrobacterium-mediated transfer, plant virus mediated transfer, microinjection, microprojectile bombardment, electroporation, PEG-mediated transformation and transformation of plant protoplasts with virus-based stable vectors.
In a preferred embodiment of the present invention said DNA sequence is contained in a vector under the control of a promoter allowing its expression in said transgenic plant.

In a particularly preferred embodiment of the present invention said vector is pHTT2-5A⁺ which has been deposited under the requirements of the Budapest treaty at the "Deutsche Sammlung für Mikroorganismen (DSM)" under the accession number DSM 6815.

In a further preferred embodiment of the present invention said introduction is carried out by transfection using the Agrobacterium system.

A further object of the present invention is the use of a genetically engineered DNA sequence encoding at least one polypeptide having a 2,5A synthetase activity, for the production of a transgenic plant displaying multiple virus resistance.
- Fig. 1:: Construction of plasmid pHTT2-5A+ . The EcoRI fragment containing the rat 2,5A synthetase gene cDNA was excised from plasmid pSK2-5A+, the cohesive termini were filled in and the obtained fragment was ligated into the BamHI site of vector pHTT202 using BamHI linkers. This results in the vector pHTT2-5A+ containing the rat 2,5A synthetase gene under the control of the CaMV 35S promoter.

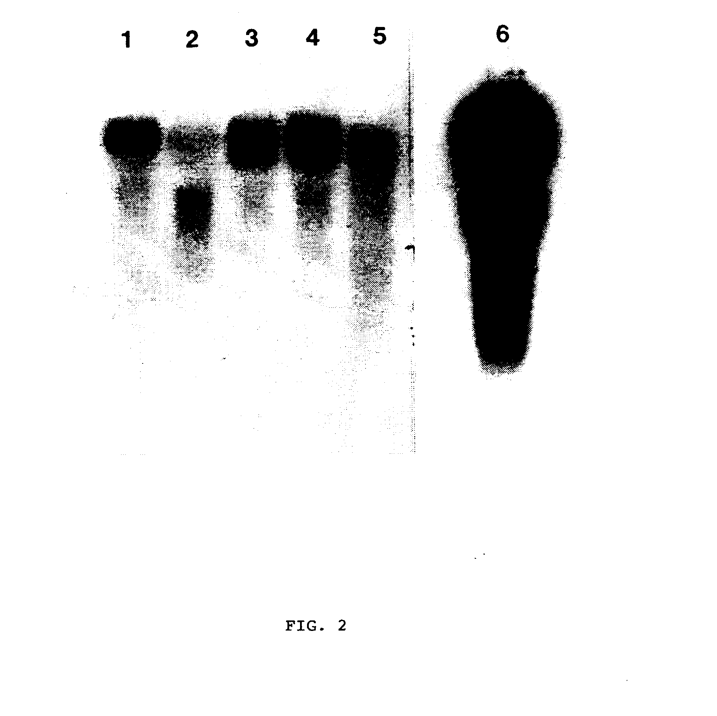

- Fig. 2:: Northern blot analysis of total RNA from transgenic tobacco plants transformed with a 2,5A synthetase gene. 10 µg of total RNA per lane were, probed with [³²P] -labelled 2,5A synthetase cDNA. Lanes 1 - 5 are transgenic tobacco lines and lane 6 is the rat 2,5A synthetase cDNA.

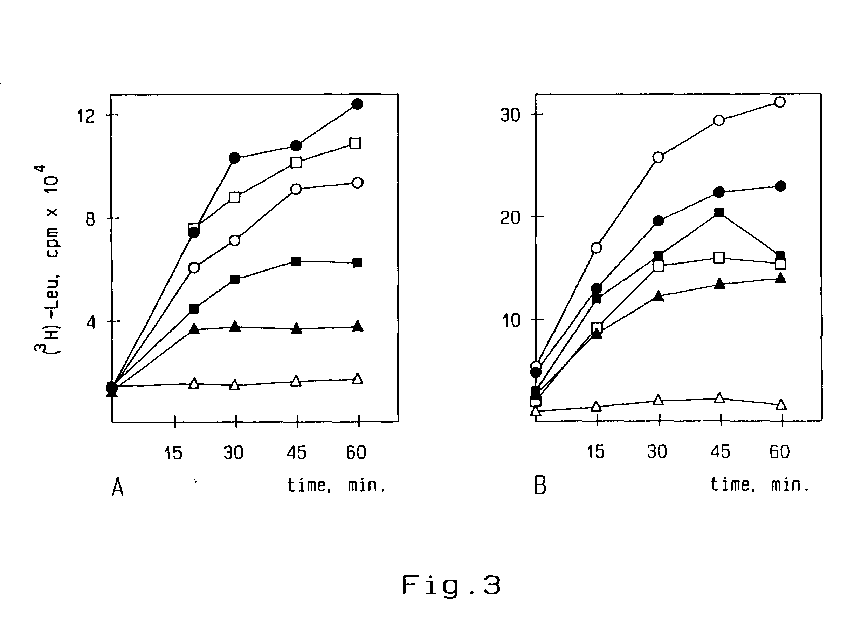

- Fig. 3:: Effect of differently phosphorylated forms of 1µm 2,5A trimers (A) and tetramers (B) on TMV RNA in vitro translation in wheat germ extract. 0.8 µg of TMV-RNA were translated in vitro, the [³⁵S]-labelled proteins obtained from 5 µl samples were precipitated on glassfiber filters and then the incorporated radioactivity was measured: ● - without 2,5A, ○ - pppAₙ, ■ - ppAₙ, □ - pAₙ, Δ - Aₙ, ▲ - without RNA.

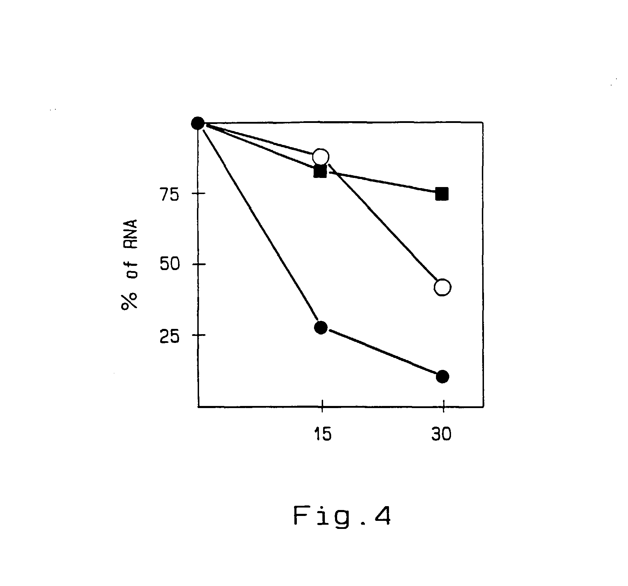

- Fig. 4:: Effect of different 2,5A trimers on TMV RNA degradation rate in wheat germ extract. TMV-RNA was isolated from samples of wheat germ extract containing 1 µM 2,5A trimers, Northern blotted and hybridized with [³²p]-labelled TMV-cDNA. The amount of RNA was estimated from autoradiographs by laser densitometer: ■ - without 2,5A, ○ - pppA₃, ● - A₃.

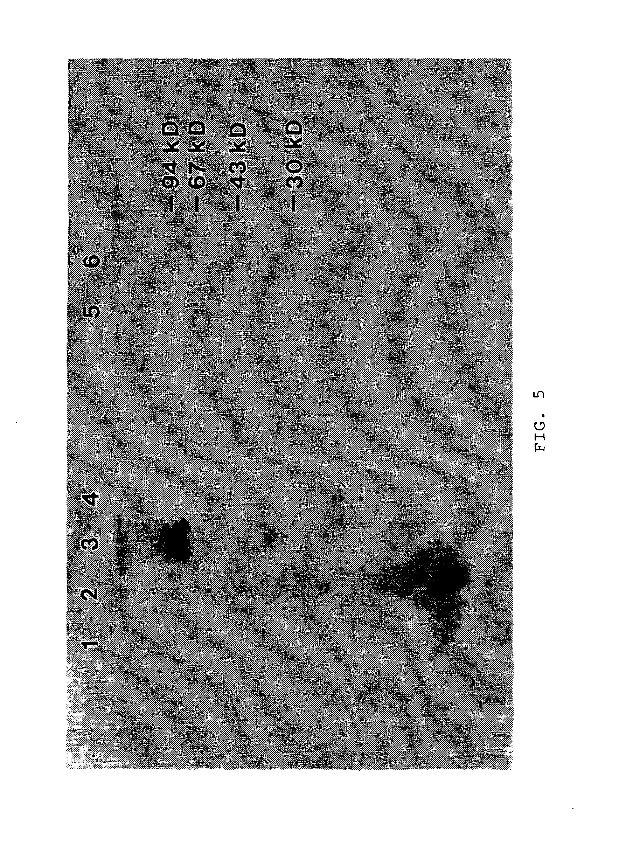

- Fig. 5:: Binding of 2,5A to plant cell extract proteins. [³²P]-labelled 2,5A was covalently crosslinked to proteins of cell extracts by the NaIO₄-oxydation method and separated on 12% SDS-PAAG electrophoresis. Lane 1 is rabbit reticulocyte lysate (prepared according to Sambrook et al., "Molecular Cloning: A Laboratory Manual", Cold Spring Harbour Laboratory, Cold Spring Harbour Laboratory Press (1989)), lane 2 is mouse L-cell extract with unlabelled 2,5A, lane 3 is mouse L-cell extract, lane 4 is rabbit reticulocyte lysate (Amersham), lane 5 is potato leaf extract and lane 6 is potato leaf extract with unlabelled 2,5A.

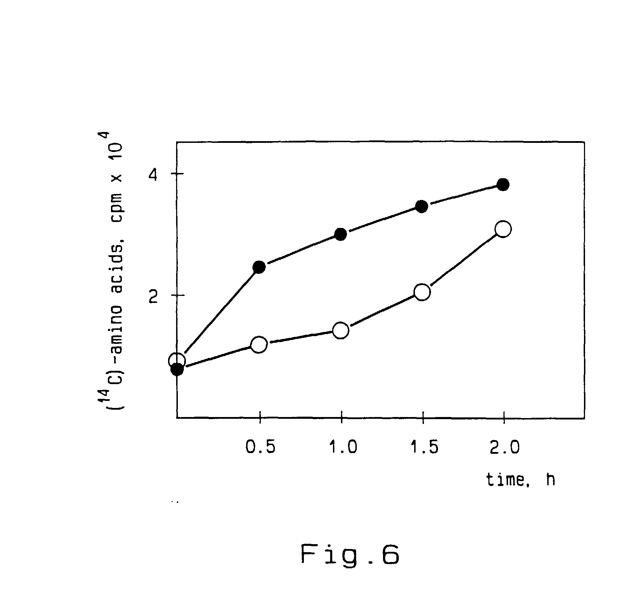

- Fig. 6:: Effect of 2,5A trimer "core" on protein synthesis in tobacco protoplasts. [¹⁴C]-labelled protein hydrolysate was added to a tobacco protoplast culture and total radioactivity of cells was measured: ● - without 2,5A, O - 1µM A₃.

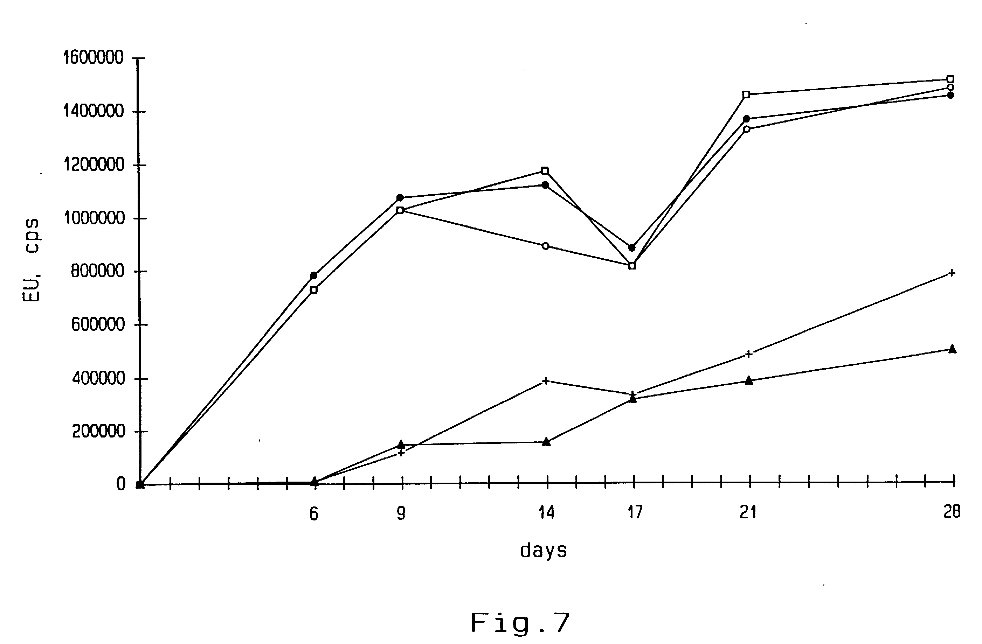

- Fig. 7:: Propagation of PVX in transgenic tobacco plants containing the 2,5A synthetase gene. Intact plants were infected with 10µg/ml PVX and leaf samples were analyzed by TRFIA using 21XD2 antibodies and 21XD2 europium conjugate. The virus concentration was determined by Eu fluorescence counts per second: ● - SR1 control, O - 4N1, □ - 4N5, + - 4N11, ∗ - 4N12.

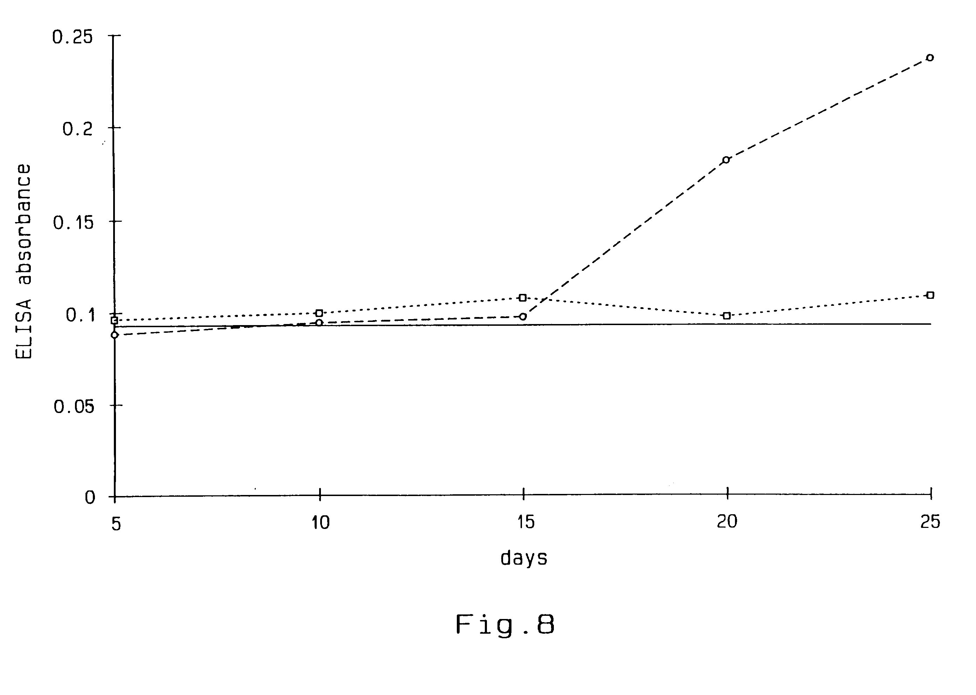

- Fig. 8:: Propagation of PVS in transgenic tobacco plants containing the 2,5A synthetase gene. Intact plants were infected with 10µg/ml PVS, and leaf samples were analyzed by ELISA using S4A4 antibodies and S4A4 horse radish peroxidase conjugate. The virus concentration is determined by optical density of colour reaction at 450 nm: O - SR1 control, □ - transgenic plants with 2,5A synthetase gene in "sense" orientation, - - ELISA background.

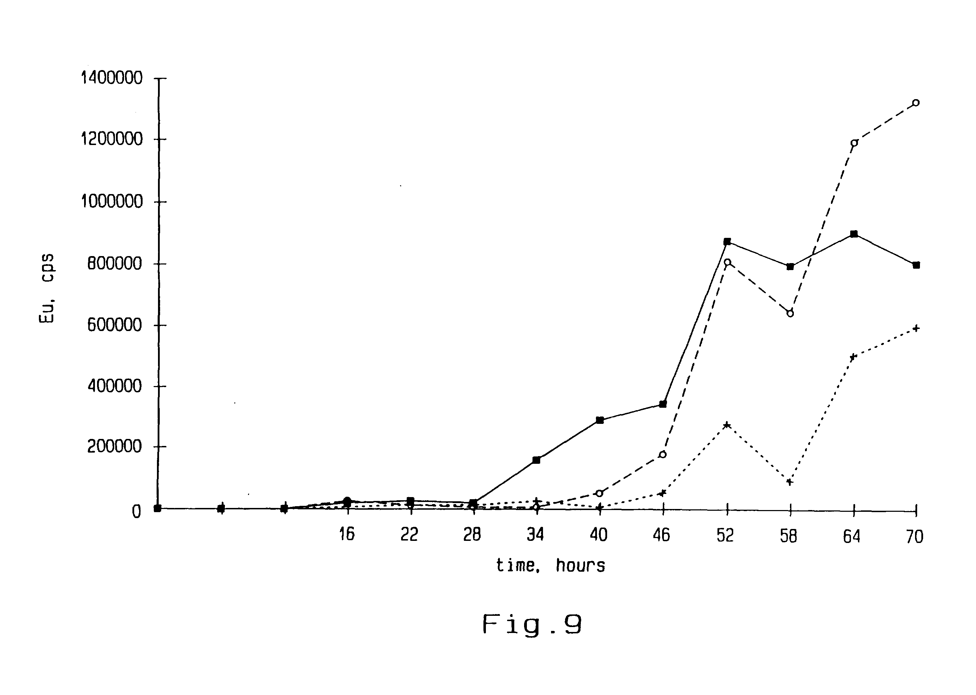

- Fig. 9:: Propagation of PVX in leaf discs of transgenic plants containing the 2,5A synthetase gene. Leaves were infected with 10µg/ml PVX, discs with 8mm diameter were punched out and kept in sterile water. The discs were analyzed as PVX-infected intact plants (see Fig. 7): ● - SR1 control, ○ - 4N10, + - 4N12.

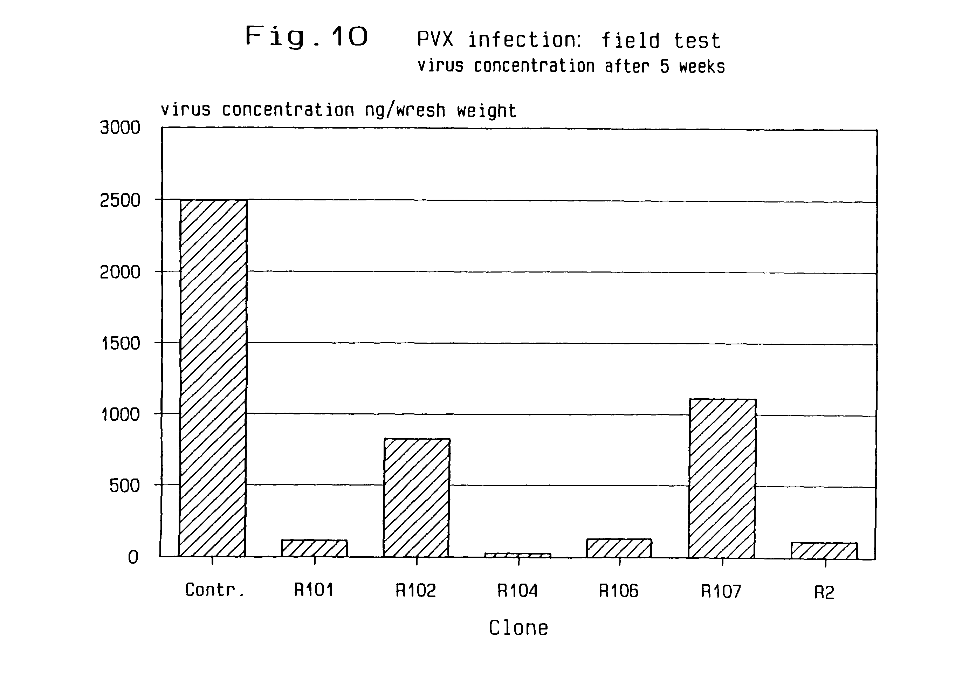

- Fig. 10:: Virus concentration (PVX) in the field grown transgenic potato plants containing 2,5A synthetase gene. Intact plants were infected with sap fluid collected from PVX infected tobacco plants. Leaf samples were analysed by ELISA, using immunodiagnostic kit for PVX (Boehringer Mannheim). The virus concentration is determined by optical density of colour reaction at 405 nm. Contr. = control clone, R101, R102, R104, R106, R107 and R2 = transgenic clones

The following examples illustrate the invention. Further explanations of the molecular biological methods applied can be found in Sambrook et al., "Molecular Cloning: A Laboratory Manual", supra.

### Example 1

### A. Construction of the 2,5A gene containing plasmid pHTT2-5A+

Rat 2,5A synthetase cDNA was isolated from a rat hippocampus cDNA library in the vector λgt10 according to Amersham protocols using mouse 2,5A synthetase cDNA as a probe. cDNA was subcloned into the EcoRI site of pBluescript SK+ (Stratagene) expression vector. The resulting plasmid was named pSK2-5A.
For transformation of tobacco plants, rat 2,5A synthetase cDNA was subcloned into the plant expression vector pHTT202. cDNA was excised from pSK2-5A by EcoRI, the cohesive termini were filled in and the cDNA was cloned into BamHI-linearized pHTT202 using synthetic BamHI linkers (Fig. 1). The resulting plasmid pHTT2-5A+ was used to transform Agrobacterium cells.

### B. Transformation of Agrobacterium with the plasmid pHTT2-5A+

The plasmid pHTT202 is largely based on plasmid pBR322 sequence. pBR322-based cloning vectors are normally not mobilized from the host cell to another bacterium but they contain the "basis of mobilization", the bom site. When "helper" functions encoded by mob genes of helper plasmids are provided, the pBR322-based cloning vectors are also mobilized. For this purpose biparental mating based on the system described by Van Haute et al., EMBO J. 2 (1983), 411-417 is used.
E. coli containing plasmid pHTT2-5A+ (amp^{R}, spc/str^{R}) was mated on L-plates with E. coli helper strain GJ23, which contains the Ia-type plasmid R64drd11 (tet^{R}, str^{R}) for the necessary tra functions and a second helper plasmid pGJ28 (kan^{R}, neo^{R}) providing mob functions in trans to complement transmission of pBR-based mob bom⁺ plasmids.
The cells with helper plasmids were selected on L-plates containing ampicillin, tetracyclin and kanamycin. These cells were cocultivated in L-broth with Agrobacterium tumefaciens strain C58C1 containing Ti-plasmid pGV2260 (rif^{R}, cb^{R}) where the genes responsible for tumorigenesis in the plant cell have been replaced by pBR322 sequences. Conjugated Agrobacterium cells were selected on plates containing rifampicin, spectinomycin and streptomycin. Since pBR322-based vectors cannot replicate in Agrobacterium, only Agrobacteria where recombination between the pBR322-sequence of pGV2260 and pHTT202 has lead to the formation of a cointegrate molecule can grow on selective plates. In order to confirm that T-DNA of recombinant plasmids contains the 2,5A synthetase gene, total DNA of resulting Agrobacteria was isolated (Dhaese et al., Nucl. Acids Res. 7, (1979), 1837-1849) and analyzed by Southern blotting.

### Example 2

### Transformation of tobacco plants with 2,5A synthetase gene

Transgenic Nicotiana tabacum SR1 plants were obtained via Agrobacterium-mediated transfer (Zambryski, Annu. Rev. Genet. 22 (1988), 1-30). The method is based on the phenomenon that Agrobacterium stably transfers a segment of its Ti-plasmid into a plant genome upon contact with wounded plant cells. The segment called "T-DNA" is defined by its conserved border sequences. The genetic information between the border sequences can be replaced without affecting the efficiency of T-DNA transfer.
Transformation of tobacco plants with the 2,5A synthetase gene was carried out according to Horsch et al., Science 227 (1985), 1229-1231. N. tabacum SR1 leaves from plants grown on MS/2 medium (Murashige and Skoog, Phys. Plant. 18 (1962), 473-497) in sterile conditions at 24°C for 16 hour photoperiods were placed upside down in K3-400 mM sucrose medium (Nagy and Maliga, Z. Pflanzenphysiol. 78 (1976), 453-455) and cut in 0.5-1 cm² pieces so that all edges were cut. Agrobacterium with cointegrate T-DNA grown in M9 minimal medium (Sambrook et al., "Molecular cloning, A Laboratory Manual" (1989), supra) was added to K3-400 mM sucrose medium and dishes were kept for three days under the same conditions as for plants. The leaf discs were washed and transferred upside down to solid LS medium (Linsmaier and Skoog, Phys. Plant. (1965), 100-127) containing Claforan to kill Agrobacteria, benzylaminopurine (BAP) to induce shoots from leaf discs and kanamycin to select for the transformed cells because the segment of plasmid pHTT2-5A+ cointegrated in T-DNA of pGV2260 also contained the nos-npt2 gene conferring kanamycin resistance to the transformed cells. After shoots appeared, they were cut and rooted on similar medium without BAP. Fully rooted plants were kept on MS/2 medium under the conditions described above.
In order to verify that plants were true transformants, total DNA and RNA from leaf tissue was isolated according to Dellaporta et al., Plant Mol. Biol. Rep. 1 (1983), 19-21 and Verwoerd et al., Nucl. Acids Res. 17 (1989), 2362, respectively. Southern and Northern blotting analysis was carried out according to Amersham protocols using [³²P]-labelled 2,5A synthetase cDNA as a probe (Fig. 2).

### Example 3

### Identification of 2,5A dependent RNase activity

Studies on the effect of different 2,5 oligoadenylate forms by tobacco mosaic virus (TMV) in vitro translation in wheat germ extract (WGE) show that nonphosphorylated tri- and tetramers of 2,5A can efficiently inhibit TMV RNA translation in WGE (Fig. 3). The degradation rates of TMV RNA during in vitro translation in the presence and absence of 2,5A were analyzed by taking samples from the translation mixture and isolating RNA as described by Toots et al., Mol. Biol. (USSR) 22 (1988), 1473-1481. After electrophoresis in agarose/formamide gel and Northern blotting, the concentration of RNA in the samples was estimated by scanning the autoradiographs of [³²P]-labelled filters with a laser densitometer. Addition of 2,5A trimer "core" led to a rapid degradation of TMV RNA, whereas the degradation rate of TMV RNA was much lower in control experiments with or without triphosphorylated 2,5A (Fig. 4). In conclusion, the inhibitory effect of the 2,5A "core" on in vitro translation in plant cell-free extract is caused by rapid degradation of substrate RNA.
In order to identify 2,5A-binding proteins in plant extracts, 2,5A was ligated to [³²P]pCp as described by Knight et al., Methods in Enzymology 79 (1981), 216-227. [³²P]-labelled 2,5A was covalently crosslinked to proteins of potato cell extracts by the NaIO₄ oxydation method according to Wreschner et al., Eur. J. Biochem. 124 (1982), 261-268. The specificity of binding was controlled in a competition assay where the unlabelled 2,5A was added in excess to reaction mixtures. The reaction mixtures were separated by 12% SDS-polyacrylamide gelelectrophoresis (SDS-PAGE).
A 70 kD protein was identified in potato leaf extracts which specifically bound to labelled 2,5A (Fig. 5). The specificity of binding was shown in the competition assay where unlabelled 2,5A completely displaced [³²P]2,5A in potato extracts (Fig. 5).
Since 2,5A-induced inhibition of in vitro translation in WGE was caused by rapid degradation of TMV RNA, the 70 kD 2,5A-binding protein from plant extract is a 2,5A-activated plant endoribonuclease.
The effect of 2,5A on protein synthesis in N. tabacum SR1 protoplasts was also tested. Protoplasts were obtained according to Honkanen et al., "Biotechnology in tropical crop improvement: proceedings of the international biotechnology workshop", (1988) from the leaf mesophyll of plants grown under sterile conditions and maintained in K3-400 mM sucrose medium. After adding [¹⁴C]-labelled amino acid hydrolysate, samples were collected from the protoplast mixture to analyze the concentration of de novo synthesized proteins. Proteins were precipitated by trichloroacetic acid and incorporated radioactivity was measured. 2,5A efficiently inhibited protein synthesis in tobacco protoplasts (Fig. 6), which again indicates the 2,5A action as a protein synthesis inhibitor in plant systems in vivo.

Having identified a 2,5A dependent RNAse activity in potato and tobacco it can be concluded that there is a metabolic pathway in plants which partially resembles the IFN-induced metabolic pathway of mammals that causes a viral resistance.

### Example 4

### Propagation of potato viruses X and S and tobacco mosaic virus in 2,5A transgenic plants

In order to examine the propagation of different viruses in transgenic plants expressing 2,5A synthetase, various infection experiments were carried out. Plants growing on MS/2 medium were transferred on soil two weeks prior to the infection. For the infection, 2-3 lower leaves were treated with carborundum, 10 µg/ml of virus were manually inoculated on the leaf surface and carborundum was washed away after 30 minutes with sterile water. Plants were kept for 16 hour photoperiods for one month. Samples were collected by taking one of the small top leaves on every fifth day and freezing it in liquid nitrogen.
Leaf samples were homogenized in a mortar adding 1 ml immunoassay buffer per gram of leaf material. For detection of potato virus X (PVX), monoclonal antibodies 21XD2 and time-resolved fluoroimmunoassay (TRFIA) technique were used as described by Sober et al., J. gen. Virol. 69 (1988), 1799-1807 and Sinijärv et al., J. gen. Virol. 69 (1988), 991-998, respectively. PVS concentration was measured by DAS-ELISA using monoclonal S4A4 antibodies (Sinijärv et al., 1988, supra).
One month postinfection with 10 µg/ml PVX, all transgenic plants contained detectable levels of PVX, but the virus concentration was much lower than in control plants (Fig. 7). Moreover, in these plants the detectable propagation of PVX started 1-2 weeks later than in control plants.
One month postinfection with 10 µg/ml PVS, all transgenic tobacco lines showed total resistance to PVS infection and contained hardly any detectable level of PVS (Fig. 8).
Leaf disc infections with PVX and additionally with TMV were also carried out. Leaves of transgenic tobaccos were infected as described above. Discs with a diameter of 8 mm were punched out of the leaves and kept upside down in sterile water at 24°C under 16 hour photoperiods for up to 5 days. The discs were frozen in liquid nitrogen and homogenized. The infection with 10 µg/ml PVX gave similar results to those with intact plants. Leaf discs of some transgenic tobacco lines contained less virus and the detectable propagation of potato viruses starts much earlier than in intact plants. The concentration of TMV in leaf discs five days postinfection showed a clear inhibition of virus propagation in respect of control plants.

### Example 5

### Detection of 2,5 oligoadenylates in transgenic plants

In order to detect the concentration of 2,5A in transgenic tobacco SR1 plants expressing 2,5A synthetase, leaf extracts from virus-infected and non-infected plants were prepared. A competition assay of unlabelled 2,5A from plant extracts with 2,5A[³²P]pCp was carried out. Mouse L-cell extract was used as a source for 2,5A-binding endoribonuclease L (RNase L). The mixture of labelled 2,5A, L-cell extract and leaf extract was incubated for 90 minutes on ice. Proteins from the mixture were precipitated on nitrocellulose filters and the radioactivity of filters and filtrate was counted. The mixture without plant extract was used as a positive control and the mixture where 10⁻⁷ M of unlabelled 2,5A trimer completely displaced the labelled 2,5A was used as a negative control.
The results show that non-infected transgenic plants expressing 2,5A synthetase contained low (if any) detectable amount of 2,5A similar to that of control plants and transgenic plants carrying 2,5A synthetase gene in antisense orientation (Table 1). This is because 2,5A synthetase is expressed as an inactive enzyme which can be activated by dsRNA. When transgenic plants expressing the 2,5A synthetase were infected with PVX, the 2,5A became detectable and its intracellular concentrations were even higher than in control mixtures. Such an increase of 2,5A was not detected in SR1 control plants. The increase of 2,5A in transgenic plants results in inhibition of PVX-, PVS- and TMV-propagation.

**Table 1**

| Competitive radiobinding assay for the determination of the concentration of 2,5 oligoadenylates in 2,5A synthetase transgenic plants pre- and postinfection with PVX | |
|---|---|
| Plants | Normalized % of the filtrate (pos. control without plant material is taken as 100%) |
| Control SR1 | 29.2 |
| Transgenic 2,5A, antisense construct | 65.5 |
| Transgenic 2,5A, sense construct | 47.2 |
| Control SR1 x PVX | 54.6 |
| Transgenic 2,5A+ x PVX | 191.7 |
| without plant extract | 100.0 |

As a conclusion, double stranded replicating intermediates of PVX RNA are able to activate the inactive 2,5A synthetase which results in the synthesis of intracellular 2,5A.

### Example 6

### Detection of PVX infection in transgenic potato plants: Field test

Intact potato plants containing the 2.5 A synthetase gene were infected with sap fluid collected from PVX infected tobacco plants and grown in the field.
Leaf samples were analysed five weeks after infection by ELISA using an immunodiagnostic kit for PVX (Boehringer Mannheim). The virus concentration was determined by optical density of colour formation at 405 nm (see Fig. 10). The concentration of PVX in plants containing the 2.5 A synthetase gene was drastically reduced as compared to control plants.

## Claims

1. A transgenic plant displaying multiple virus resistance, which contains a genetically engineered DNA sequence encoding at least one polypeptide having a 2,5A synthetase activity, wherein said polypeptide upon expression is capable of activating an endonuclease causing degradation of viral RNA.

2. The transgenic plant according to claim 1, wherein said DNA sequence is a heterologous DNA sequence.

3. The transgenic plant according to claim 1 or 2, wherein said DNA sequence is a chimeric DNA sequence.

4. The transgenic plant according to any one of claim 1 to 3, wherein the coding sequence of said polypeptide is derived from a mammalian gene, a plant gene or a microorganism gene or is a synthetic gene.

5. The transgenic plant according to any one of claim 1 to 4, wherein the coding sequence of said polypeptide is a rat 2,5A synthetase gene.

6. The transgenic plant according to any one of claims 1 to 5, wherein the expression of said polypeptide is under the control of an inducible or a constitutive promoter.

7. The transgenic plant according to any one of claims 1 to 6, which is tobacco, rice, wheat, barley, maize, tomato, cucumber, soya, sweet potato, grapes, rapeseed, sugar beet, cotton, tea, sunflower, strawberry, rose, chrysanthemum, sweet pepper or potato.

8. Transgenic propagating material derived from a transgenic plant according to any one of claims 1 to 7.

9. A process for the production of a transgenic plant according to any one of claims 1 to 7, comprising the introduction of a genetically engineered DNA sequence encoding at least one polypeptide having a 2,5A synthetase activity into the genetic material of a suitable plant.

10. The process according to claim 9, wherein said DNA sequence is contained in a vector under the control of a promoter allowing its expression in said transgenic plant.

11. The process according to claim 10, wherein said vector is pHTT2-5A+ (DSM No. 6815).

12. The process according to any one of claims 9 to 11, wherein said introduction is carried out by transfection using the Agrobacterium system.

13. Use of a genetically engineered DNA sequence encoding at least one polypeptide having a 2,5A synthetase activity for the production of a transgenic plant according to any one of claims 1 to 7.

## Patentansprüche

1. Multiple Virus-Resistenz aufweisende transgene Pflanze, die eine unter Verwendung gentechnischer Verfahren hergestellte DNA-Sequenz enthält, die mindestens ein Polypeptid mit einer 2,5A-Synthetaseaktivität codiert, wobei das Polypeptid nach Expression in der Lage ist, eine Endonuclease zu aktivieren, die den Abbau von viraler RNA bewirkt.

2. Transgene Pflanze gemäß Anspruch 1, wobei die DNA-Sequenz eine heterologe DNA-Sequenz ist.

3. Transgene Pflanze gemäß Anspruch 1 oder 2, wobei die DNA-Sequenz eine chimäre DNA-Sequenz ist.

4. Transgene Pflanze gemäß einem der Ansprüche 1 bis 3, wobei die codierende Sequenz des Polypeptids von einem Säugergen, einem Pflanzengen oder einem Mikroorganismengen abgeleitet ist oder ein synthetisches Gen ist.

5. Transgene Pflanze gemäß einem der Ansprüche 1 bis 4, wobei die codierende Sequenz des Polypeptids ein Ratten-2,5A-Synthetasegen ist.

6. Transgene Pflanze gemäß einem der Ansprüche 1 bis 5, wobei die Expression des Polypeptids unter der Kontrolle eines induzierbaren oder konstitutiven Promotors steht.

7. Transgene Pflanze gemäß einem der Ansprüche 1 bis 6, die Tabak, Reis, Weizen, Gerste, Mais, Tomate, Gurke, Soja, Süßkartoffel, Weintraube, Raps, Zuckerrübe, Baumwolle, Tee, Sonnenblume, Erdbeere, Rose, Chrysantheme, Paprika oder Kartoffel ist.

8. Transgenes Vermehrungsmaterial, abgeleitet von einer transgenen Pflanze gemäß einem der Ansprüche 1 bis 7.

9. Verfahren zur Herstellung einer transgenen Pflanze gemäß einem der Ansprüche 1 bis 7, umfassend die Einführung einer unter Verwendung gentechnischer Verfahren hergestellten DNA-Sequenz, die mindestens ein Polypeptid mit 2,5A-Synthetaseaktivität codiert, in das genetische Material einer geeigneten Pflanze.

10. Verfahren gemäß Anspruch 9, wobei die DNA-Sequenz in einem Vektor enthalten ist unter der Kontrolle eines Promotors, der ihre Expression in der transgenen Pflanze erlaubt.

11. Verfahren gemäß Anspruch 10, wobei der Vektor pHTT2-5A+ (DSM Nr. 6815) ist.

12. Verfahren gemäß einem der Ansprüche 9 bis 11, wobei die Einführung durch Transfektion mittels dem Agrobacterium-System durchgeführt wird.

13. Verwendung einer unter Verwendung gentechnischer Verfahren hergestellten DNA-Sequenz, die mindestens ein Polypeptid mit einer 2,5A-Synthetaseaktivität codiert, zur Herstellung einer transgenen Pflanze gemäß einem der Ansprüche 1 bis 7.

## Revendications

1. Plante transgénique manifestant une résistance à plusieurs virus, qui contient une séquence d'ADN génétiquement modifiée codant pour au moins un polypeptide ayant une activité 2,5A-synthétase, dans laquelle ledit polypeptide après expression est capable d'activer une endonucléase provoquant une dégradation d'ARN viral.

2. Plante transgénique selon la revendication 1, dans laquelle ladite séquence d'ADN est une séquence d'ADN hétérologue.

3. Plante transgénique selon la revendication 1 ou 2, dans laquelle ladite séquence d'ADN est une séquence d'ADN chimère.

4. Plante transgénique selon l'une quelconque des revendications 1 à 3, dans laquelle la séquence codante dudit polypeptide est issue d'un gène mammalien, d'un gène de plante ou d'un gène de micro-organisme, ou est un gène synthétique.

5. Plante transgénique selon l'une quelconque des revendications 1 à 4, dans laquelle la séquence codante dudit polypeptide est un gène de 2,5A-synthétase de rat.

6. Plante transgénique selon l'une quelconque des revendications 1 à 5, dans laquelle l'expression dudit polypeptide est sous le contrôle d'un promoteur inductible ou d'un promoteur constitutif.

7. Plante transgénique selon l'une quelconque des revendications 1 à 6, qui est le tabac, le riz, le blé, l'orge, le maïs, la tomate, le concombre, le soja, la patate douce, le raisin, le colza, la betterave à sucre, le coton, le théier, le tournesol, le fraisier, le rosier, le chrysanthème, le poivron ou la pomme de terre.

8. Matériel de propagation transgénique issu d'une plante transgénique selon l'une quelconque des revendications 1 à 7.

9. Procédé pour la production d'une plante transgénique selon l'une quelconque des revendications 1 à 7, comprenant l'introduction d'une séquence d'ADN génétiquement modifié codant pour au moins un polypeptide ayant une activité 2,5A-synthétase, dans le matériel génétique d'une plante appropriée.

10. Procédé selon la revendication 9, dans lequel ladite séquence d'ADN est contenue dans un vecteur sous le contrôle d'un promoteur permettant son expression dans ladite plante transgénique.

11. Procédé selon la revendication 10, dans lequel ledit vecteur est pHTT2-5A+ (n°DSM 6815).

12. Procédé selon l'une quelconque des revendications 9 à 11, dans lequel ladite introduction est effectuée par transfection à l'aide du système *Agrobacterium.*

13. Utilisation d'une séquence d'ADN génétiquement modifiée codant pour au moins un polypeptide ayant une activité 2,5A-synthétase, pour la production d'une plante transgénique selon l'une quelconque des revendications 1 à 7.
